# DEMANDE DE BREVET EUROPEEN

(11) **EP 4 684 829 A1**
(43) Date de publication de la demande: **28.01.2026**
(21) Numéro de dépôt: 25189141.2
(22) Date de dépôt: 11.07.2025
(51) Int. Cl.: A61N 5/06

(54) **DISPOSITIF POUR EXPOSER LA PEAU D'UN UTILISATEUR À UN RAYONNEMENT LUMINEUX OU INFRAROUGE**

(30) Priorité: 22.07.2024 FR 2408035
(71) Demandeur: Xbiotec, 38110 Saint-Clair-de-la-Tour (FR)
(72) Inventeur: HOUOT, Jean-Laurent, 38110 Saint-Clair-de-la-Tour (FR); LEROY, Sebastien, 38110 La chapelle de la Tour (FR)
(74) Mandataire: IXAS Conseil

(57) **Abrégé**

Un dispositif pour exposer la peau d'un utilisateur à un rayonnement lumineux ou infrarouge, qui comporte au moins une source de rayonnement comportant une tête (130, 230) comportant au moins une diode électroluminescente, ladite tête étant configurée pour être fixée en place au contact de la peau,
le dispositif comportant en outre au moins un câble (150) configuré pour être raccordé avec la tête d'une part et pour être raccordé avec une base (180) d'autre part et
la base (180) comportant une source de courant et/ou un moyen de commande, de sorte que le raccordement entre la base et l'au moins une source de rayonnement par l'au moins un câble permet l'alimentation électrique de l'au moins une diode électroluminescente et/ou le transfert de données informatiques commandant l'intensité lumineuse émise par l'au moins une diode électroluminescente.

## Description

### DOMAINE TECHNIQUE DE L'INVENTION

La présente invention concerne un dispositif pour exposer la peau d'un utilisateur à un rayonnement lumineux ou infrarouge. Ce dispositif est utilisé dans des traitements de photothérapie, c'est-à-dire d'exposition de l'utilisateur à la lumière à des fins thérapeutiques. Selon un mode particulier, le dispositif est prévu pour traiter l'utilisateur par exposition de points d'acupuncture à un rayonnement lumineux ou infrarouge.

### ÉTAT DE LA TECHNIQUE

L'art antérieur connaît des pratiques et dispositifs visant à stimuler les points traditionnellement stimulés dans le cadre d'un traitement d'acupuncture mais sans recourir à des aiguilles. Ces pratiques incluent l'acupression, qui consiste en des pressions exercées par le thérapeute, l'acupuncture par stylet qui consiste en l'utilisation d'un appareil de stimulation électrique et l'acupuncture par laser, qui consiste en l'utilisation d'une source de rayonnement laser manipulée à la main par le praticien qui vient stimuler les points d'acupuncture dans le but d'obtenir un effet thérapeutique, telle qu'une réduction de la douleur.

Un avantage revendiqué de ces alternatives à l'acupuncture est d'obtenir des résultats équivalents à ceux de l'acupuncture mais sans l'usage d'aiguilles, qui peuvent ne pas être appréciées par la personne traitée ou poser un risque d'infection. Ces méthodes présentent toutefois l'inconvénient que les points d'acupuncture doivent être stimulés un par un lors d'une séance de traitement. Par exemple dans le cas d'une acupuncture par laser, le praticien tient à la main la source de rayonnement laser et doit l'appliquer tour à tour sur chacun des points prévus lors du traitement, contrairement à un traitement traditionnel d'acupuncture, qui permet de stimuler une multitude de points simultanément en plaçant autant d'aiguilles d'acupuncture que nécessaire. Alternativement, le praticien peut recourir à un dispositif comportant un émetteur de rayonnement laser raccordé à une pluralité de fibres optiques, mais dans ce cas il n'est pas possible de moduler pour chaque fibre la puissance et la fréquence du rayonnement émis. De plus, ces systèmes tendent à être peu pratiques, car la multitude de fibres optiques, par exemples 15 à 20 fibres, prennent de la place et s'emmêlent.

La société demanderesse a identifié qu'il existait un besoin d'offrir un nouveau type de dispositif de traitements de photothérapie, par exemple pour un traitement alternatif à l'acupuncture, mais également utile pour la médecine douce du sport.

### OBJETS DE L'INVENTION

La présente invention vise à offrir un dispositif amélioré de traitements d'un utilisateur par photothérapie. Selon un mode particulier, l'invention vise à fournir un dispositif pour réaliser un traitement alternatif à l'acupuncture. La présente invention vise notamment à offrir un dispositif plus polyvalent et plus maniable que ceux disponibles sur le marché, permettant par exemple de fixer des sources de rayonnement lumineux sur la peau de l'utilisateur, préférentiellement en plusieurs points simultanément. La présente invention vise à titre secondaire un système de capuchon sanitaire permettant une utilisation pratique du dispositif dans des conditions optimales d'hygiène.

À cet effet, selon un premier aspect, l'invention vise un dispositif pour exposer la peau d'un utilisateur à un rayonnement lumineux ou infrarouge, qui comporte au moins une source de rayonnement,
l'au moins une source de rayonnement comportant une tête comportant au moins une diode électroluminescente, ladite tête étant configurée pour être fixée en place au contact de la peau,
le dispositif comportant en outre au moins un câble configuré pour être raccordé avec la tête d'une part et pour être raccordé avec une base d'autre part et ladite base comportant une source de courant et/ou un moyen de commande, de sorte que le raccordement entre la base et l'au moins une source de rayonnement par l'au moins un câble permet l'alimentation électrique de l'au moins une diode électroluminescente et/ou le transfert de données informatiques commandant l'intensité lumineuse émise par l'au moins une diode électroluminescente.

La mise en œuvre de diodes électroluminescentes qui sont bien plus compactes et légères qu'une source de rayonnement laser permet de fournir une source de rayonnement compacte, d'une taille et d'un poids suffisamment réduit pour permettre à la source de rayonnement d'être fixée en place au contact de la peau. Ainsi le praticien peut déposer la ou les sources de rayonnement sur la peau de l'utilisateur et les fixer au moyen d'un ruban adhésif. Grâce à ces dispositions, des traitement nécessitant la stimulation d'un point de la peau sur une période prolongée, par exemple de quelques minutes, pourront être mis en œuvre. Préférentiellement, une pluralité de sources de rayonnement sont positionnées et fixées en place en différents points de la peau de l'utilisateur, afin de stimuler lesdits différents points simultanément.

Préférentiellement, la source de rayonnement comporte une unique diode électroluminescente, ce qui permet d'obtenir une source de rayonnement particulièrement compacte.

Dans des modes de réalisation, la tête est amovible et comporte un port de connexion configuré pour raccorder le câble avec la tête amovible.

Selon ce mode de réalisation les têtes des sources de rayonnement sont amovibles, ce qui présente un avantage en termes de polyvalence. En effet, des têtes amovibles de plusieurs types, ayant des formes différentes adaptées à différents traitements et/ou des diodes électroluminescentes émettant des rayonnements dans des longueurs d'ondes différentes pourrons être fournies au praticien. Ainsi, une même base et des mêmes câbles pourront être utilisées avec différentes têtes amovibles, afin de réaliser des traitements différents.

Par exemple, une tête amovible particulière présente une forme adaptée et comporter une pince, de sorte à être fixée sur l'oreille d'un utilisateur. Par exemple une tête amovible, telle que décrite dans la présente demande, est adaptée pour être accolée contre la peau. Selon un autre exemple, la tête amovible présente la forme d'un pad, c'est-à-dire d'un élément en matière souple de forme sensiblement plate et configuré pour être apposé contre la peau. Un tel pad pourra comporter une pluralité de diodes électroluminescentes, par exemple entre 2 et 6 diodes et pourra présenter de dimension de l'ordre de 5 à 20 centimètres de hauteur, de 4 à 10 centimètres de largeur et de 0,5 à 3 centimètres d'épaisseur.

Dans des modes de réalisation, le dispositif comporte une pluralité de têtes et l'au moins un câble comporte :
- un segment primaire de câble comportant une extrémité configurée pour être raccordée avec la base,
- une pluralité de segments secondaires de câble issus de la subdivision du segment primaire
et chaque segment secondaire est doté d'une extrémité configurée pour être raccordée avec le port de connexion d'une tête amovible.

Grâce à ces dispositions, l'encombrement des câbles est réduit. Par exemple un même câble comportant quatre segments secondaires, chacun configuré pour être raccordé à une tête, remplace quatre câbles. Cette configuration facilite le travail du praticien sans nuire à la bonne tenue du traitement. En effet, plusieurs points d'acupuncture se trouvent dans la même zone du corps et doivent habituellement être stimulés en même temps.

Dans des modes de réalisation, l'au moins une source de rayonnement comporte une surface configurée pour être positionnée au contact de la peau de l'utilisateur et au moins un diffuseur de lumière configuré pour transmettre la lumière émise par l'au moins une diode électroluminescente vers la peau de l'utilisateur, ledit diffuseur formant un relief par rapport à ladite surface.

Ainsi, le diffuseur une fois positionné sur la peau lorsque la source de rayonnement est fixée en place, par exemple au moyen d'un ruban adhésif, appuie légèrement contre la peau de l'utilisateur. Ces dispositions permettent de concentrer l'exposition lumineuse en un point précis de la peau et de s'assurer qu'il n'y ait pas d'espace entre le diffuseur et la surface de la peau lors de l'utilisation. Lorsqu'un tel espace existe, la lumière se diffuse dans l'air et l'intensité lumineuse reçue par la peau est moindre. Le diffuseur en saillie de la surface configurée pour être positionnée au contact de la peau permet d'éviter cet effet de diffusion.

Dans des modes de réalisation, au moins une source de rayonnement comporte un capuchon sanitaire configuré pour recouvrir au moins en partie la surface de la tête amovible. Par exemple le capuchon sanitaire et est un pochon en plastique souple et transparent dans lequel est inséré la tête de la source de rayonnement.

Grâce à ces dispositions, la transmission de pathogène d'un utilisateur à l'autre peut être évitée en remplaçant le capuchon sanitaire entre deux utilisations successives.

Dans des modes de réalisation, le capuchon sanitaire est un élément rigide. Préférentiellement, la source de rayonnement comporte un moyen de fixation réversible du capuchon sanitaire sur la tête. Par exemple, le capuchon sanitaire est un capuchon sanitaire en matière en plastique dont la forme est complémentaire ou correspond à celle de la tête, de sorte que le capuchon et la tête peuvent être maintenus en place. Préférentiellement un moyen de fixation telle qu'un clip, un crochet ou une attache magnétique est prévu pour maintenir le capuchon fixé à la tête.

Avantageusement le capuchon rigide est un capuchon réutilisable configuré pour être lavé entre deux utilisations.

Dans des modes de réalisation, le moyen de fixation comporte au moins une encoche et au moins un relief, prévus sur le capuchon sanitaire et sur la tête amovible, configurés pour coopérer entre eux pour établir une fixation par clipsage entre le capuchon sanitaire et la tête amovible.

Grâce à ces dispositions, la tête et le capuchon sanitaire peuvent être fixés de manière réversible l'un à l'autre. On souligne que le relief et l'encoche peuvent respectivement être formés sur le capuchon sanitaire et la tête amovible, ou inversement.

Dans des modes de réalisation, la tête amovible comporte une fibre optique configurée pour conduire le rayonnement lumineux ou infra-rouge émis par la diode électroluminescente.

Dans des modes de réalisation, le port de connexion est un port USB, préférentiellement un connecteur USB de type-A ou un connecteur USB-C.

Selon un deuxième aspect, l'invention vise une source de rayonnement pour exposer un corps humain ou animal à un rayonnement lumineux ou infrarouge, qui comporte une tête amovible comportant au moins une diode électroluminescente et un port de connexion,
ledit port de connexion étant configuré pour être relié par l'intermédiaire d'un câble à une base comportant une source de courant et/ou un moyen de commande, de sorte que le raccordement entre la base et la source de rayonnement par le câble permet l'alimentation électrique de la diode électroluminescente et/ou le transfert de données informatiques commandant l'intensité lumineuse émise par la diode électroluminescente.

Dans d'autres modes de réalisation, les caractéristiques techniques décrites ci-avant concernant la source de rayonnement du dispositif objet de l'invention peuvent être mises en œuvre pour la source de rayonnement objet de l'invention. En particulier, la ou les source de rayonnement pourront être associé à un câble subdivisé en une pluralité de segments secondaires, la ou les source de rayonnement pourront comporter des têtes amovibles et la ou les source de rayonnement pourront comporter des capuchons sanitaires.

Les buts, avantages et caractéristiques particulières de la source de rayonnement objet de la présente invention étant similaires à ceux du dispositif objet de la présente invention, ils ne sont pas rappelés ici.

### BRÈVE DESCRIPTION DES FIGURES

D'autres avantages, buts et caractéristiques particulières de l'invention ressortiront de la description non limitative qui suit d'au moins un mode de réalisation particulier du dispositif et de la source de rayonnement pour exposer un corps humain ou animal à un rayonnement lumineux ou infrarouge objets de la présente invention, en regard des dessins annexés, dans lesquels :
[Fig 1] représente, schématiquement, une vue en perspective d'un mode de réalisation particulier d'un dispositif objet de l'invention.
[Fig 2] représente, schématiquement, un détail de la figure 1, centré sur l'extrémité du câble reliée aux têtes amovibles.
[Fig 3] représente, schématiquement et en vue en perspective, un premier mode de réalisation particulier d'une source de rayonnement, pouvant être mise en œuvre dans un dispositif objet de l'invention.
[Fig 4] représente, schématiquement et en vue en perspective, un deuxième mode de réalisation particulier d'une source de rayonnement, pouvant être mise en œuvre dans un dispositif objet de l'invention.
[Fig 5] représente, schématiquement et en vue en perspective, un troisième mode de réalisation particulier d'une source de rayonnement, pouvant être mise en œuvre dans un dispositif objet de l'invention.
[Fig 6] représente, le troisième mode de réalisation d'une source de rayonnement mais représenté sous un autre angle, schématiquement et en vue en perspective.

Les numéros de référence mentionnés sur les figures se rapportent à :
10 Dispositif pour exposer un corps à un rayonnement
130, 230, 330 tête amovible
132, 232, 332 connexion USB-C femelle
135, 235, 335 coque de la tête amovible
236 fibre optique
136, 336 diffuseur
138, 238, 338 surface de contact
339 cavité du clip
340 capuchon sanitaire
343 bordure
344 trou borgne
349 relief du clip
150 segment primaire de câble
155 pièce intermédiaire
161, 162, 163, 164 segment secondaire de
171, 172, 173, 174 Connexion USB-C mâle
180 Base
190, 192 Connexion USB type-A sur la base
195 Écran tactile

### DESCRIPTION DÉTAILLÉE DE L'INVENTION

La présente description est donnée à titre non limitatif, chaque caractéristique d'un mode de réalisation pouvant être combinée à toute autre caractéristique de tout autre mode de réalisation de manière avantageuse.

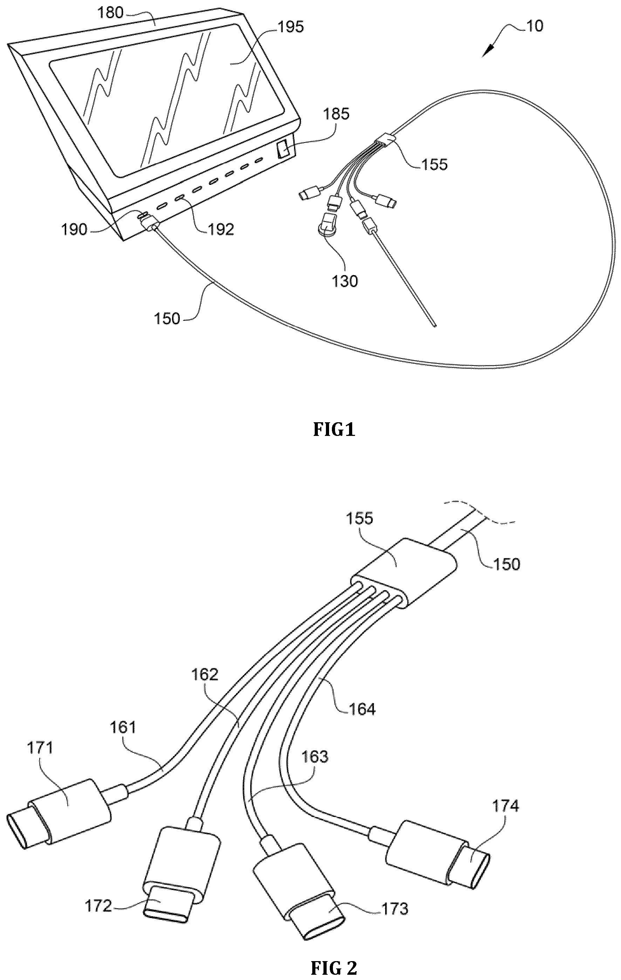

On observe, sur la figure 1 une vue schématique et en perspective d'un mode de réalisation particulier d'un dispositif 10 pour exposer un corps humain ou animal à un rayonnement lumineux ou infrarouge. La figure 2 représente quant à elle un détail de la figure 1, centré sur une extrémité du câble visible en figure 1.

Le dispositif 10 comporte une base 180. La base comporte une interface homme-machine permettant la commande du dispositif 10 par l'opérateur et la commande du fonctionnement du dispositif 10. L'affichage des données de fonctionnement du dispositif 10 sont affichées sur un écran 195, ces données incluent par exemple l'intensité lumineuse émise par chacune de sources de rayonnement du dispositif et le temps d'exposition restant ou déjà écoulé. L'écran 195 est préférentiellement un écran tactile et sert d'interface homme-machine.

On précise à ce stade que, dans la présente demande le terme « utilisateur » est utilisé pour désigner la personne sur laquelle est réalisée le traitement de photothérapie et on utilise le terme « opérateur » pour désigner la personne qui manipule le dispositif objet de l'invention pour réaliser le traitement de photothérapie. L'utilisateur et l'opérateur peuvent être une seule et même personne lorsque la personne réalise un traitement sur elle-même.

La base 180 comporte un interrupteur 185 et une pluralité de ports 190 de type USB type-A qui sont chacun configurés pour être connecté à une extrémité d'un câble 150. La base 180 comporte un ou plusieurs câbles configurés pour être raccordé avec le port de connexion d'au moins une tête amovible 130, 230 d'une part et pour être raccordé avec la base 180 d'autre part.

La base 180 comporte une source de courant et un moyen de commande, de sorte que le raccordement entre la base 180 et au moins une source de rayonnement par l'intermédiaire d'un câble permet l'alimentation électrique d'une diode électroluminescente montée sur la source de rayonnement et le transfert de données informatiques commandant l'intensité lumineuse émise par ladite diode électroluminescente. Avantageusement, le moyen de commande est configuré pour commander l'intensité lumineuse émise par chaque diode électroluminescente (LED) ou groupe de LED. Dans des modes de réalisation, les LED émettent des flashs lumineux, dans ce cas le moyen de commande est configuré pour commander la fréquence des flashs lumineux et leur durée.

Préférentiellement chaque câble comporte un segment primaire 150 de câble comportant une extrémité configurée pour être reliée électriquement à la base 180 et une pluralité de segments secondaires 161, 162, 163, 164 de câble issus de la subdivision du segment primaire et rattachés par une pièce intermédiaire 155 qui permet de dissimuler la distribution des fils électriques entre les segments secondaires. Chaque segment secondaire 161, 162, 163, 164 de câble est doté d'une extrémité 171, 172, 173, 174 configurée pour être raccordée avec le port de connexion, 132, 232, 332, d'une tête amovible 130, 230, 330.

Le dispositif 10 comporte une ou préférentiellement plusieurs sources de rayonnement configurée pour être positionnée au contact de la peau d'un utilisateur. Dans un mode de réalisation non illustré une source de rayonnement comporte une diode électroluminescente fixée de manière permanente à l'extrémité d'un câble, subdivisé ou non et segments secondaires.

L'au moins une diode électroluminescente (LED) est préférentiellement configurée pour émettre un rayonnement dans une gamme de longueur d'onde comprise entre 400 nanomètres (nm)et 950 nm. Par exemple, tout ou partie des LED sont configurées pour émettre un rayonnement en lumière bleue ayant une longueur d'onde comprise entre 450 nm et 490 nm, en lumière rouge ayant une longueur d'onde située entre 600 nm et 670 nm ou un rayonnement en lumière proche infrarouge ayant une longueur d'onde située entre 820 nm et 860 nm.

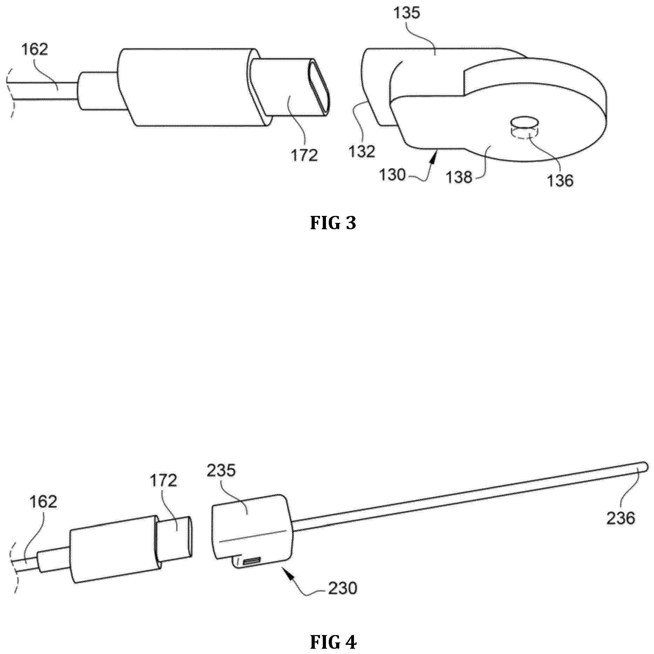

Préférentiellement et comme illustré sur les figures 1, 3, 4, 5 et 6. La source de rayonnement comporte une tête amovible 130, 230, 330 comportant au moins une diode électroluminescente et un port de connexion 132, 232, 332. À des fins d'illustration, une tête amovible 130 configurée pour plaquée contre la peau d'un utilisateur et une tête amovible 230 comportant une fibre optique sont représentée en figure 1 en position pour être branchées avec l'extrémité subdivisée du câble. On note cependant qu'en condition normale d'utilisation ce seront habituellement des têtes amovibles du même type qui seront utilisées. Les caractéristiques des têtes amovibles seront mieux comprises à la lecture de la description des figures 3, 4, 5 et 6.

On observe en figure 3 un premier mode de réalisation particulier d'une source de rayonnement comportant une tête amovible 130. La tête amovible 130 comporte un port de connexion 132 de type USB-C femelle configuré pour être raccordée électriquement au port de connexion 172, de type USB-C mâle, d'un segment de câble 162.

Les composants interne de la tête amovible, qui incluent au moins une diode électroluminescente sont protégés par une coque 135, préférentiellement en matière plastique. La tête amovible est de petite taille de sorte à pouvoir être facilement manipulée et placée contre la peau de l'utilisateur et de sorte à pouvoir être fixée en place à l'aide d'un ruban adhésif. Les dimensions de la tête amovible sont par exemple comprises entre 2 et 5 centimètres (cm) de longueur, comprise entre 2 et 4 cm de largeur et comprises entre 1 et 4 centimètres de hauteur. Le poids de la tête amovible 130 n'excède préférentiellement pas 150 grammes, préférentiellement ce poids n'excède pas 120 grammes.

La tête amovible comporte une surface 138 sensiblement plane, qui est configurée pour être plaquée contre la peau de l'utilisateur. Avantageusement, un diffuseur de lumière 136 transmettant la lumière depuis une diode électroluminescente dépasse en saillie de la surface 138 sensiblement plane, de sorte à former un relief. Ainsi, lorsque la surface 138 est plaqué contre la peau, elle obture la lumière extérieure. De plus, la position en saillie du diffuseur 136 permet un contact direct entre le diffuseur et la peau, ce qui assure une meilleure transmission de la lumière vers la peau, sans déperdition dans une couche d'air qui séparerait le diffuseur de la peau.

Avantageusement, la face de la tête amovible opposée à la face comportant la surface 138 sensiblement plane configurée pour être apposée contre la peau de l'utilisateur est également sensiblement plane ou forme un arrondi. Ainsi, le collage d'une bande adhésive sur la tête amovible qui relie la tête amovible à la peau de l'utilisateur est facilité.

On observe en figure 4 un deuxième mode de réalisation particulier d'une source de rayonnement comportant une tête amovible 230. La tête amovible 230 comporte un port de connexion de type USB-C femelle configuré pour est raccordé électriquement à un port de connexion d'un câble 162 de type USB-C mâle.

La tête amovible 230 comporte diode électroluminescente abritée par une coque 235 en matière plastique. La tête amovible 230 comporte en outre une fibre optique 236 dont une extrémité est accolée à la diode électroluminescente, de sorte à transmettre la lumière qu'elle émet vers l'autre extrémité. Lors de l'utilisation d'un dispositif objet de l'invention avec de telles tête amovible, la fibre optique est positionnée en des points prédéterminés de la peau de l'utilisateur. Une bande adhésive ou tout autre moyen de fixation peut être utiliser pour maintenir en place la fibre optique contre la peau de l'utilisateur.

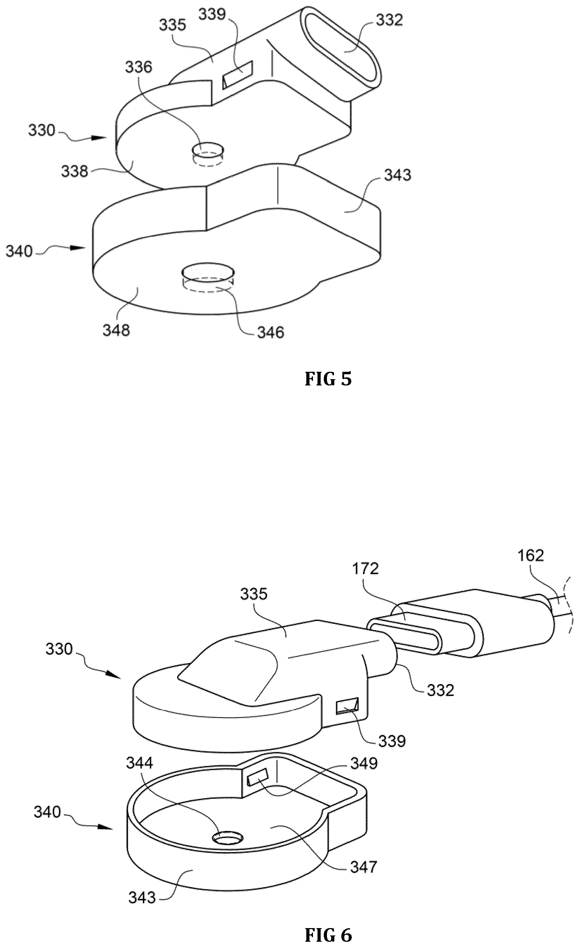

On observe en figure 5 et en figure 6 un troisième mode de réalisation particulier d'une source de rayonnement comportant une tête amovible 330. La tête amovible 330 comporte un port de connexion 332, une coque 335, une surface 338 et un diffuseur 336 qui sont respectivement similaires aux éléments 232, 235, 238 et 236 décrits en regard de la figure 3.

La source de rayonnement illustrée en figure 5 et 6 se distingue de celle illustrée en figure 3 en ce qu'elle est associée à un capuchon sanitaire 340 configuré pour recouvrir au moins en partie la surface de la tête amovible. Le capuchon sanitaire est amovible et configuré pour être réversiblement attaché à la tête 330 le temps d'un traitement pour un utilisateur. Le capuchon sanitaire est ensuite enlevé et éventuellement remplacé par un autre pour le prochain utilisateur. Préférentiellement, le capuchon sanitaire 340 est en matière plastique. Avantageusement, la coque 335 comporte deux encoches 339 aptes à coopérer avec deux reliefs 349 prévus sur le capuchon sanitaire 340 de sorte à clipser ensemble la tête 330 et le capuchon sanitaire 340.

Le capuchon sanitaire 340 se présente préférentiellement sous la forme d'un récipient ouvert et à fond plat, comportant une surface formant le fond du récipient et comportant une bordure 343 configurée pour présenter une forme complémentaire à celle de la tête amovible. Ladite surface est comporte deux faces, une première face 347 configuré pour être accolée à la surface 338 sensiblement plane sur la tête amovible330 et une deuxième face 348 prévue pour être posée contre la peau de l'utilisateur.

Le capuchon sanitaire comporte une partie transparente alignée avec le diffuseur 336 de la tête amovible lorsque la tête amovible et le capuchon sanitaire sont montés l'un sur l'autre. Dans des modes de réalisation, le capuchon sanitaire dans son ensemble est formé dans un plastique transparent.

Préférentiellement, le capuchon sanitaire comporte un trou borgne 344 positionné en alignement avec le diffuseur 336 ressortant en saillie de la tête amovible 330. La taille du trou borgne 344 est adaptée pour abriter le diffuseur 336 de sorte que le diffuser 336 est logé dans le trou borgne 344 lorsque la tête amovible et le capuchon sanitaire sont montés l'un sur l'autre.

Avantageusement, le capuchon sanitaire comporte un relief 348 sur la deuxième face 348 prévue pour être posée contre la peau de l'utilisateur. Le relief 348 est formé par la face opposé à la face forment le fond du trou borgne 344. Le relief 348 et formé dans une matière transparente et configuré pour transmettre la lumière émise par la diode électroluminescente. Le relief 348 dépasse en saillie par rapport à la surface 348, permettant ainsi une meilleure diffusion de la lumière depuis la diode électroluminescente vers la peau de l'utilisateur, en évitant qu'une couche d'air ne soit présente.

## Revendications

1. Dispositif (10) pour exposer la peau d'un utilisateur à un rayonnement lumineux ou infrarouge, **caractérisé en ce qu'**il comporte au moins une source de rayonnement,
l'au moins une source de rayonnement comportant une pluralité de têtes (130, 230, 330) comportant au moins une diode électroluminescente, lesdites têtes étant configurées pour être fixée en place au contact de la peau,
lesdites têtes étant amovibles et comportant un port de connexion (132, 232, 332)
le dispositif comportant en outre au moins un câble (150) configuré pour être raccordé avec lesdites têtes d'une part et pour être raccordé avec une base (180) d'autre part l'au moins un câble comportant :
- un segment primaire (150) de câble comportant une extrémité configurée pour être raccordée avec la base,
- une pluralité de segments secondaires (161, 162, 163, 164) de câble issus de la subdivision du segment primaire,
et chaque segment secondaire étant doté d'une extrémité (171, 172, 173, 174) configurée pour être raccordée avec le port de connexion d'une tête (130, 230, 330)et
la base (180) comportant une source de courant et/ou un moyen de commande, de sorte que le raccordement entre la base et l'au moins une source de rayonnement par l'au moins un câble permet l'alimentation électrique de l'au moins une diode électroluminescente et/ou le transfert de données informatiques commandant l'intensité lumineuse émise par l'au moins une diode électroluminescente.

2. Dispositif selon la revendication 1, dans lequel au moins une source de rayonnement comporte une surface (138, 348) configurée pour être positionnée au contact de la peau de l'utilisateur et au moins un diffuseur de lumière (336, 346) configuré pour transmettre la lumière émise par l'au moins une diode électroluminescente vers la peau de l'utilisateur, ledit diffuseur formant un relief par rapport à ladite surface.

3. Dispositif selon l'une des revendications 1 ou 2, dans lequel au moins une source de rayonnement comporte un capuchon sanitaire configuré pour recouvrir au moins en partie la surface de la tête.

4. Dispositif selon la revendication précédente, dans lequel le capuchon sanitaire est un élément rigide et dans lequel la source de rayonnement comporte un moyen de fixation du capuchon sanitaire sur la tête.

5. Dispositif selon la revendication précédente, dans lequel ledit moyen de fixation comporte au moins une encoche (339) et au moins un relief (349), prévus sur le capuchon sanitaire et sur la tête amovible, configurés pour coopérer entre eux pour établir une fixation par clipsage entre le capuchon sanitaire et la tête.

6. Dispositif selon l'une des revendications 1 à 5, dans lequel la tête comporte une fibre optique configurée pour conduire le rayonnement lumineux ou infra-rouge émis par la diode électroluminescente.

7. Dispositif selon l'une des revendications 1 à 6, dans lequel le port de connexion est un port USB (Universal Serial Bus), préférentiellement un connecteur USB de type-A ou un connecteur USB-C.

8. Source de rayonnement pour exposer un corps humain ou animal à un rayonnement lumineux ou infrarouge, **caractérisé en ce qu'**elle comporte une pluralité de têtes (130, 230, 330) comportant au moins une diode électroluminescente et configurée pour être relié par l'intermédiaire d'un câble (150) à une base (180) comportant une source de courant et/ou un moyen de commande, de sorte que le raccordement entre la base et la source de rayonnement par le câble permet l'alimentation électrique de la diode électroluminescente et/ou le transfert de données informatiques commandant l'intensité lumineuse émise par la diode électroluminescente
lesdites têtes étant amovibles et comportant un port de connexion (132, 232, 332)
ledit câble comportant :
- un segment primaire (150) de câble comportant une extrémité configurée pour être raccordée avec la base,
- une pluralité de segments secondaires (161, 162, 163, 164) de câble issus de la subdivision du segment primaire,
chaque segment secondaire étant doté d'une extrémité (171, 172, 173, 174) configurée pour être raccordée avec le port de connexion d'une tête (130, 230, 330).

9. Source de rayonnement selon la revendication 8, qui comporte une surface (138, 348) configurée pour être positionnée au contact de la peau de l'utilisateur et au moins un diffuseur de lumière (336, 346) configuré pour transmettre la lumière émise par l'au moins une diode électroluminescente vers la peau de l'utilisateur, ledit diffuseur formant un relief par rapport à ladite surface.

10. Source de rayonnement selon l'une des revendications 8 ou 9, qui comporte un capuchon sanitaire configuré pour recouvrir au moins en partie la surface de la tête.

11. Source de rayonnement selon la revendication précédente, dans lequel le capuchon sanitaire est un élément rigide et dans lequel la source de rayonnement comporte un moyen de fixation du capuchon sanitaire sur la tête.

12. Source de rayonnement selon la revendication précédente, dans lequel ledit moyen de fixation comporte au moins une encoche (339) et au moins un relief (349), prévus sur le capuchon sanitaire et sur la tête amovible, configurés pour coopérer entre eux pour établir une fixation par clipsage entre le capuchon sanitaire et la tête.

13. Source de rayonnement selon l'une des revendications 8 à 12, dans lequel la tête comporte une fibre optique configurée pour conduire le rayonnement lumineux ou infra-rouge émis par la diode électroluminescente.

14. Source de rayonnement selon l'une des revendications 8 à 13, dans lequel le port de connexion est un port USB (Universal Serial Bus), préférentiellement un connecteur USB de type-A ou un connecteur USB-C.
